# EUROPEAN PATENT APPLICATION

(11) **EP 4 414 375 A1**
(43) Date of publication of application: **14.08.2024**
(21) Application number: 22878817.0
(22) Date of filing: 30.09.2022
(51) Int. Cl.: C07K 5/113, A61K 38/00, A61P 29/00, A61K 8/64, A61Q 19/00

(54) **PEPTIDE HAVING ANTI-INFLAMMATORY ACTIVITY AND USE THEREOF**

(30) Priority: 05.10.2021 KR 20210131401
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); KIM, Seon Soo, Anyang-si, Gyeonggi-do 14119 (KR)
(74) Representative: Heinonen & Co
(86) International application number: PCT/KR2022/014734
(87) International publication number: WO 2023/058996

(57) **Abstract**

The present invention relates to a novel peptide having anti-inflammatory activity and to a use of the peptide for preventing, treating, or improving inflammatory diseases on the basis of an inflammation suppressing effect. More specifically, the novel peptide according to the present invention inhibits the expression or secretion of a gene or protein that promotes an inflammatory response and thus has the advantage that the novel peptide can be used as an active ingredient of a pharmaceutical composition for preventing or treating inflammatory diseases that are caused by or accompany the inflammatory response, or can be used as an active ingredient of a health functional food or cosmetic composition for preventing or relieving inflammatory diseases.

## Description

### TECHNICAL FIELD

The present invention relates to a novel peptide having anti-inflammatory activity and an inflammation inhibiting effect and use thereof.

### BACKGROUND ART

Inflammation, which is a type of defense reaction of living tissue that occurs when tissue or cells are damaged or injured or infected by an external infectious agent (viruses, bacteria, fungi, allergens, *etc*.), refers to a series of complex conditions caused by immune cells involved in various immune responses that gather around the center and the inflammatory factors they secrete. Inflammation is a lesion that causes three things: tissue degeneration, circulatory failure and exudation, and tissue proliferation, and in general, it is initiated and maintained by the chemotaxis of neutrophils among white blood cells. Major factors involved in the inflammatory response include histamine secreted by mast cells, interferon gamma (IFNγ) secreted by T cells or NK cells, various interleukins secreted by macrophages, nitric oxide (NO), prostaglandins, *etc*.

In principle, since inflammation is a defense reaction of the living body, it acts to regenerate the tissue that has been injured by the inflammation or to restore damaged functions of the living body by removing external infectious agents; however, when the external infectious agents are not completely removed or continuous and excessive inflammation occurs due to internal substances, various human diseases may be caused by the above-mentioned abnormal inflammation, and it is also associated with diseases such as autoimmune diseases and cancer. In particular, representative examples include acute inflammation occurring within joints, diseases such as rheumatoid arthritis, skin diseases such as psoriasis, and allergic inflammation such as bronchial asthma.

It is possible to find ways to treat or improve inflammatory diseases by stopping inflammation by way of inhibiting the activity of cells involved in causing or maintaining inflammation or inhibiting the production of inflammation-inducing substances or enzymes secreted by the same. With the development of molecular biology, cytokines that cause inflammatory diseases are being understood at the molecular level, and studies have been conducted to identify inflammation-related factors, and efforts have been made to develop therapeutic agents by attempting to inhibit inflammation by inhibiting the expression or activity of these cytokines.

Currently known therapeutic agents for inflammatory diseases include dexamethasone, cortisone, etc. which use adrenocortical hormone components; however, although these therapeutic agents have activity as therapeutic agents, they are highly toxic and may cause side effects such as edema. In addition, it has been reported that since these therapeutic agents do not have a selective effect on the cause of inducing inflammation, a severe level of immune inhibition may occur, which may cause problems. As described above, since the attempts of treating inflammatory diseases using drugs containing steroid ingredients are accompanied by side effects and problems, there is an urgent need for the development of a therapeutic agent for inflammatory diseases which can stably exhibit an anti-inflammatory treatment effect without side effects using non-steroidal drugs.

### [Prior Art Document]

### [Patent Document]

Patent Document 1: Korean Patent Application Publication No. 10-2020-0043476
Patent Document 2: Korean Patent Application Publication No. 10-2017-0124472

### DISCLOSURE OF THE INVENTION

### TECHNICAL PROBLEM

An object of the present invention is to provide a peptide which has anti-inflammatory activity and is effective in inhibiting the expression of inflammatory cytokines and inflammatory marker proteins such as Cox2 and iNos, and thus may be used to inhibit inflammation.

Additionally, the object of the present invention is to provide a pharmaceutical composition for preventing or treating inflammatory diseases, including the peptide as an active ingredient.

Additionally, the object of the present invention is to provide a health functional food for preventing or improving inflammatory diseases, including the peptide as an active ingredient.

Additionally, the object of the present invention is to provide a cosmetic composition for preventing or improving inflammatory diseases, including the peptide as an active ingredient.

In order to achieve the above objects, an aspect of the present invention provides a peptide having anti-inflammatory activity, including the amino acid sequence of SEQ ID NO: 1.

Another aspect of the present invention provides a pharmaceutical composition for preventing or treating inflammatory diseases, including a peptide including the amino acid sequence of SEQ ID NO: 1 as an active ingredient.

Still another aspect of the present invention provides a health functional food for preventing or improving inflammatory diseases, including a peptide including the amino acid sequence of SEQ ID NO: 1 as an active ingredient.

Still another aspect of the present invention provides a cosmetic composition for preventing or improving inflammatory diseases, including a peptide including the amino acid sequence of SEQ ID NO: 1 as an active ingredient.

Hereinafter, the present invention will be described in detail.

### 1. Peptides having anti-inflammatory activity

An aspect of the present invention provides a novel peptide having anti-inflammatory activity.

The peptide refers to a polymer consisting of two or more amino acids linked by a peptide bond. Since peptides have disadvantages in that their sizes are too large to effectively enter target tissues or cells, or their half-lives are too short and thus disappear in the body within a short period of time, the peptides of the present invention consist of 20 or less amino acids, for example, 15 or less amino acids, or 10 or less amino acids, with anti-inflammatory activity.

The peptide of the present invention may include the amino acid sequence of SEQ ID NO: 1 and may be amino acid variants or fragments having different sequences due to deletion, insertion, substitution, or combination of amino acid residues, within the range not affecting the anti-inflammatory activity. Amino acid exchanges at the peptide level that do not overall alter the anti-inflammatory activity of the peptide are known in the art. In some cases, it may be modified by phosphorylation, sulfation, acrylation, glycosylation, methylation, farnesylation, *etc*. Therefore, the present invention includes a peptide including an amino acid sequence substantially identical to the peptide including the amino acid sequence of SEQ ID NO: 1, and a variant or active fragment thereof. The substantially identical protein refers to an amino acid sequence having sequence homology of more than 75% or more, for example, more 80% or more, 90% or more, and 95% or more, respectively, with the amino acid sequence of SEQ ID NO: 1. Additionally, the peptide may additionally include an amino acid sequence prepared for the specific purpose of a targeting sequence, a tag, a labeled residue, and increasing the half-life or stability of a peptide.

Additionally, the peptide of the present invention may be obtained by various methods well known in the art. As an example, the peptide may be prepared using polynucleotide recombination and protein expression systems, or the peptide may be prepared by *in vitro* synthesis through chemical synthesis such as peptide synthesis and cell-free protein synthesis.

Additionally, a protecting group may be attached to the N-terminus or C-terminus of the peptide in order to obtain improved chemical stability, enhanced pharmacological properties (half-life, absorptivity, potency, efficacy, *etc*.), altered specificity (*e.g*., broad spectrum of biological activity), and reduced antigenicity. For example, the protecting group may be an acetyl group, fluorenyl methoxy carbonyl group, formyl group, palmitoyl group, myristyl group, stearyl group, or polyethylene glycol (PEG), but any ingredient that can modify the peptide, especially improving the stability of the peptide, may be included without limitation. The "stability" refers not only to *in vivo* stability, which protects the peptide of the present invention from attack by proteinases *in vivo,* but also to storage stability (*e.g*., storage stability at room temperature).

The anti-inflammatory activity of the present invention refers to inhibition of inflammation, and the inflammation refers to damage of tissue or cells as a type of defense response of living tissue to a certain stimulus or a pathological condition of an abscess that is formed when infected with various infectious agents (*e.g*., bacteria, fungi, viruses, allergens, *etc*.) derived from the outside. Additionally, since the inflammation refers to complex physiological responses (*e.g*., activation of enzymes, secretion of inflammatory mediators, body fluid infiltration, cell migration, tissue destruction, *etc*.) and external symptoms (*e.g*., erythema, swelling, fever, pain, *etc*.) that occur due to association of inflammatory mediators and immune cells in local blood vessels and body fluids. Since the peptide of the present invention has anti-inflammatory activity, it provides the effect of reducing and improving a series of pathological conditions and symptoms as described above.

Additionally, the peptide of the present invention may inhibit the expression of inflammatory cytokines. When a wound occurs due to inflammation or an external infectious agent which has invaded the wound area enters the body, white blood cells responsible for the initial immune response gather around the wound or infectious agent, and express and secrete inflammation-related cytokines thus inducing inflammation, the peptide can exhibit anti-inflammatory activity by inhibiting the expression of inflammatory cytokines. Additionally, by confirming the expression levels of the inflammatory cytokine, the anti-inflammatory activity and inflammation inhibiting effect of the peptide of the present invention can be confirmed.

The inflammatory cytokine may be one or more selected from the group consisting of TNFα, IL-6, IL-17, IL-1β, and IFNγ. The TNFα, which is an abbreviation for "tumor necrosis factor α", is a cytokine produced and secreted by macrophages and various cells activated during immune responses to bacterial infections or tumor diseases, is known as a major mediator of inflammation, and plays an important role in inflammatory diseases (*e.g*., rheumatoid arthritis (RA), psoriatic arthritis, Crohn's disease, psoriasis, and ankylosing spondylitis (AS)). The interleukin 6 (IL-6) is a cytokine produced by macrophages and various lymphocytes that promotes inflammation, and is known to cause inflammatory diseases when produced excessively. The interleukin 17 (IL-17) also corresponds to a pro-inflammatory cytokine, and is produced from Th17 cells and plays a role in causing or mediating inflammation. The interferon γ (IFNγ) can be produced in T lymphocytes and macrophages, is secreted in response to infection by viruses or bacteria which have invaded from the outside, and is known to play a role in autoimmune or autoinflammatory diseases. Therefore, the peptide of the present invention has the effect of inhibiting inflammation through its activity of inhibiting the expression of inflammatory cytokines as described above and inhibiting the secretion of expressed cytokines.

The peptide of the present invention can inhibit the expression of cyclooxygenase 2 (Cox2). The Cox2 is an enzyme that participates in the biosynthetic process of prostaglandins by stimulation of the same. The Cox2 can be expressed being regulated by NF-κB and controls inflammation. Cox2 is hardly expressed under normal conditions, but is a protein that is rapidly expressed by stimulation, such as cytokines, inflammatory factors, and endotoxin, and the anti-inflammatory activity of the peptide of the present invention can be confirmed by checking the expression level of the Cox2 gene or the amount of Cox2 protein, and the peptide of the present invention has the effect of inhibiting inflammation by inhibiting the expression of Cox2.

Additionally, the peptide of the present invention can inhibit the expression of inducible nitric oxide synthase (iNos). The iNos is an enzyme that catalyzes the production of nitric oxide (NO), and in particular, the secretable form of iNos is known to be involved in immune responses. Nitric oxide excessively produced by iNos within immune cells may cause cell damage, and expression of iNos as described above can be induced from stimulation of macrophages by LPS. Since iNos, as in Cox2, is a protein involved in the regulation of inflammation, the anti-inflammatory activity of the peptide of the present invention can be confirmed by checking the expression level or protein amount of iNos, and the peptide of the present invention can inhibit and improve inflammation by inhibiting the expression of iNos.

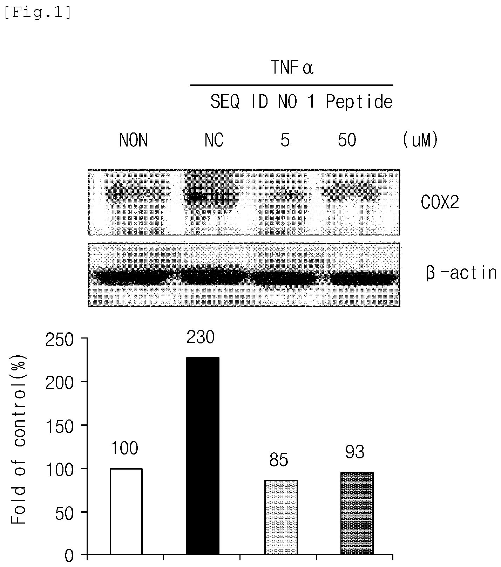

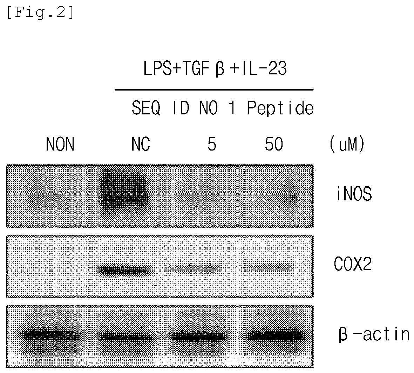

In order to confirm the anti-inflammatory effect of the peptide of the present invention, in a specific embodiment of the present invention, human keratinocytes (HaCaT) were treated with TNFα, which is an inflammation-inducing cytokine, along with a peptide including the amino acid sequence of SEQ ID NO: 1, or TGFβ, IL-23 (*i.e*., a cytokine inducing Th17 type inflammation) and LPS,, which is an inflammation-inducing antigen,and checked the amount of Cox2 and iNos proteins. As a result, it was confirmed that although inflammation of cells was induced by the treated inflammation-inducing factors, the amounts of Cox2 and iNos proteins were decreased according to the treatment with the peptide of the present invention (see FIGS. 1 and 2).

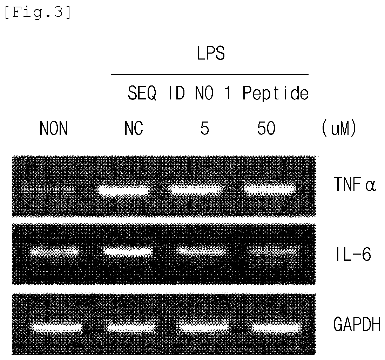

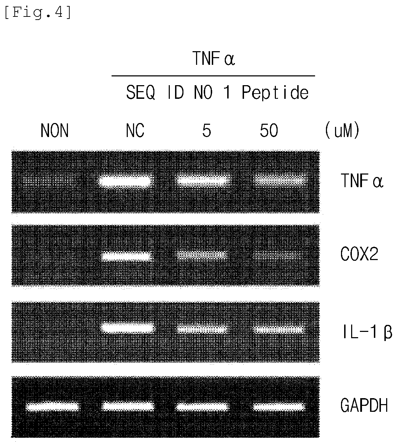

Additionally, in order to confirm the effect of reducing the expression level of inflammation-related genes according to the treatment of the peptide of the present invention, in a specific embodiment of the present invention, mouse splenocytes were treated with LPS, which is an inflammation-inducing antigen, or TNFα, which is an inflammation-inducing cytokine, together with a peptide including the amino acid sequence of SEQ ID NO: 1, and the mRNA amounts of TNFα, IL-6, Cox2, and IL-1β genes were checked. As a result, it was confirmed that although inflammation of cells was induced by the treated inflammation-inducing factors, the expression levels of inflammation-inducing cytokines or related enzyme genes described above were decreased by the treatment of the peptide of the present invention (see FIGS. 3 and 4).

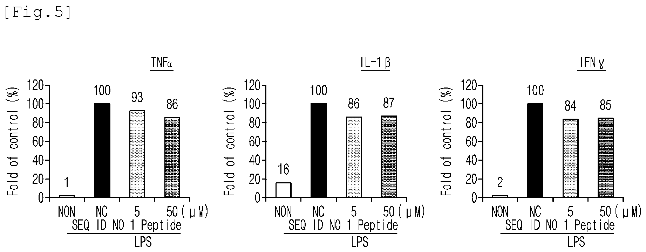

Moreover, in order to confirm the effect of reducing the secretion of inflammation-related cytokines by the treatment of the peptide of the present invention, in a specific embodiment of the present invention, mouse splenocytes were treated with LPS, which is an inflammation-inducing antigen, or TNFα, TGFβ, and IL-23 (*i.e*., inflammation-inducing cytokines) together with a peptide including the amino acid sequence of SEQ ID NO: 1, and the secretion amounts of IL-17, IL-1β, and IFNγ were checked using the ELISA technique. As a result, it was confirmed that although inflammation of cells was induced by the treated inflammation-inducing factors, the secretion of the pro-inflammatory cytokines described above was reduced upon treatment with the peptide of the present invention (see FIGS. 5 to 7).

Therefore, it is apparent that the peptide of the present invention has anti-inflammatory activity that can reduce and improve inflammation by reducing the expression or secretion of inflammatory cytokines (*e.g*., TNFα, IL-6, IL-17, IL-1β, and IFNγ, which can promote inflammation) and inhibiting the expression of inflammation-related factors (*e.g*., Cox2 and iNos), and thus, the peptide of the present invention can be usefully used as an active ingredient in a composition for preventing, treating, or improving inflammatory diseases caused by inflammation or accompanying inflammation.

### 2. Pharmaceutical composition for preventing or treating inflammatory diseases

Another aspect of the present invention provides a pharmaceutical composition for preventing or treating inflammatory diseases including a peptide including the amino acid sequence of SEQ ID NO: 1 as an active ingredient.

Since the peptide including the amino acid sequence of SEQ ID NO: 1 is the same as the peptide described in the "1. Peptides having anti-inflammatory activity" section above, for detailed explanation, the "1. Peptides having anti-inflammatory activity" section is used, and hereinafter, only the constitution specific to the pharmaceutical composition for preventing or treating inflammatory diseases will be described.

Since the peptide of the present invention has the effect of inhibiting the expression or secretion of inflammation-related cytokines or inflammation-related factors, a pharmaceutical composition including the above peptide as an active ingredient can inhibit the expression of TNFα, IL-6, IL-17, IL-1β, IFNγ, Cox2, and iNos, and thus can be used to prevent or treat inflammatory diseases.

The inflammatory disease refers to a pathological condition that causes inflammation caused by chemotaxis of neutrophils among white blood cells, and any disease caused by or accompanied by inflammation may be included. For example, the inflammatory disease may be rhinitis, bronchitis, periodontitis, pancreatitis, gastritis, gastric ulcer, inflammatory skin disease, atopic dermatitis, encephilitis, sepsis, inflammatory enteritis, chronic obstructive pulmonary disease, septic shock, pulmonary fibrosis, undifferentiated spondyloarthropathy, undifferentiated arthropathy, arthritis, inflammatory osteolysis, chronic inflammatory disease caused by chronic viral or bacterial infection, colitis, inflammatory bowel disease, type 1 diabetes, rheumatoid arthritis, reactive arthritis, osteoarthritis, psoriasis, scleroderma, osteoporosis, atherosclerosis, myocarditis, endocarditis, pericarditis, cystic fibrosis, Hashimoto's thyroiditis, Graves' disease, leprosy, syphilis, lyme, borreliosis, neuroborreliosis, tuberculosis, sarcoidosis, lupus, discoid lupus, chilblains lupus, lupus nephritis, systemic lupus erythematosus, macular degeneration, uveitis, irritable bowel syndrome, Crohn's disease, Sjögren's syndrome, fibromyalgia, chronic fatigue syndrome, chronic fatigue immune dysfunction syndrome, myalgic encephalomyelitis, amyotrophic lateral sclerosis, Parkinson's disease and multiple sclerosis, but is not limited thereto.

The pharmaceutical composition of the present invention may be used to prevent worsening of conditions and to treat diseases by inhibiting the expression or secretion of factors that cause inflammation to thereby prevent the occurrence of inflammatory diseases, or by inhibiting inflammation that occurs additionally in damaged or scarred cells of patients with the above disease.

Meanwhile, the pharmaceutical composition including the peptide of the present invention as an active ingredient may be prepared in unit dose form or by placing it in a multi-dose container by formulating the same using pharmaceutically acceptable carriers and/or excipients according to a method that can easily be performed by those skilled in the art to which the present invention pertains. In particular, the formulation may be in the form of a solution, suspension, or emulsion in an oil or aqueous medium, or may be in the form of an extract, powder, granule, tablet, capsule, or gel (*e.g*., hydrogel), and may additionally include a dispersant or stabilizer.

Additionally, the peptide included in the pharmaceutical composition may be delivered in a pharmaceutically acceptable carrier, such as colloidal suspensions, powders, salines, lipids, liposomes, microspheres, or nanospherical particles. These carriers may form a complex or associate with the vehicle, or may be delivered *in vivo* using delivery systems known in the art, such as lipids, liposomes, microparticles, gold, nanoparticles, polymers, condensation agents, polysaccharides, polyamino acids, dendrimers, saponins, adsorption enhancing materials, or fatty acids.

In addition, the pharmaceutically acceptable carrier may include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propyl hydroxybenzoate, talc, magnesium stearate, mineral oil, etc., which are commonly used in preparation, but is not limited thereto. Additionally, in addition to the above ingredients, lubricants, humectants, sweeteners, flavoring agents, emulsifiers, suspending agents, preservatives, etc. may additionally be included.

The pharmaceutical composition according to the present invention may be administered orally or parenterally during clinical administration and may be used in the form of a common pharmaceutical preparation. That is, the pharmaceutical composition of the present invention may be administered in various oral and parenteral dosage forms during actual clinical administration, and when formulated, the pharmaceutical composition is prepared using diluents or excipients, such as commonly used fillers, extenders, binders, humectants, disintegrants, and surfactants. Solid preparations for oral administration include tablets, pills, powders, granules, capsules, *etc*., and these solid preparations are prepared by mixing a herbal extract or fermented herbal medicine with at least one or more excipients, such as starch, calcium carbonate, sucrose or lactose, and gelatin. Additionally, in addition to simple excipients, lubricants such as magnesium stearate, talc are also used. Liquid preparations for oral administration include suspensions, oral solutions, emulsions, syrups, *etc*., and various excipients (*e.g*., humectants, sweeteners, fragrances, preservatives, *etc*.) may be included in addition to the commonly used simple diluents (*e.g*., water and liquid paraffin). Preparations for parenteral administration include sterilized aqueous solutions, non-aqueous solutions, suspensions, emulsions, freeze-dried preparations, and suppositories. As non-aqueous solvents and suspension solvents, propylene glycol, polyethylene glycol, vegetable oil (*e.g*., olive oil), and injectable esters (*e.g*., ethyl oleate), *etc*. may be used. As a base for suppositories, Witeppsol, Macrogol, Tween 61, cacao butter, laurinum, glycerol, gelatin, *etc*. may be used.

The pharmaceutical composition of the present invention is administered in a pharmaceutically effective amount. In the present invention, the "pharmaceutically effective amount" refers to an amount sufficient to treat the disease with a reasonable benefit/risk ratio applicable to medical treatment, and the effective dose level may be determined based on factors including the type and severity of the patient's disease, activity of the drug, sensitivity to the drug, time of administration, route of administration and excretion rate, duration of treatment, and drugs used simultaneously, and other factors well known in the medical field. The pharmaceutical composition according to the present invention may be administered as an individual therapeutic agent or in combination with other therapeutic agents for inflammatory diseases, and may be administered simultaneously with, separately from, or sequentially with conventional therapeutic agents, and may be administered once or multiple times. Considering all of the above factors, it is important to administer an amount that can achieve the maximum effect with the minimum amount without side effects, and this can easily be determined by those skilled in the art.

Specifically, the effective amount of the pharmaceutical composition may vary depending on the patient's age, sex, condition, body weight, degree of absorption of the active ingredient in the body, inactivation rate, excretion rate, type of disease, and drugs administered in combination, may increase or decrease depending on the route of administration, severity, sex, body weight, age, etc., in an embodiment, the pharmaceutical composition may be administered in an amount of about 0.0001 µg to about 500 mg, for example, 0.01 µg to 100 mg, per 1 kg of the patient's body weight per day. Additionally, the pharmaceutical composition may be administered in divided doses several times a day, for example, two to three times a day, at regular time intervals, depending on the judgment of a physician or pharmacist.

### 3. Health functional food for preventing or improving inflammatory diseases

Still another aspect of the present invention provides a health functional food for preventing or improving inflammatory diseases including a peptide including the amino acid sequence of SEQ ID NO: 1 as an active ingredient.

Since the peptide including the amino acid sequence of SEQ ID NO: 1 is the same as the peptide described in the "1. Peptides having anti-inflammatory activity" section above, for detailed description, the "1. Peptides having anti-inflammatory activity" section is used, and hereinafter, only the unique constitution of health functional foods for preventing or improving inflammatory diseases will be described.

As in the pharmaceutical composition, inflammatory diseases may be prevented or improved by inhibiting inflammation, and the health functional food including the peptide of the present invention as an active ingredient, which inhibits inflammation by inhibiting expression or secretion of inflammatory cytokines (*e.g*., TNFα, IL-6, IL-17, IL-1β, IFNγ, *etc*.) or inflammation-related proteins (*e.g.*, Cox2, iNos, *etc*.), may be effectively used in preventing or improving inflammatory diseases.

The health functional food may be used simultaneously with or separately from drugs for treatment before or after the occurrence of the disease in order to prevent or improve the disease.

In the health functional food of the present invention, the active ingredient may be added as-is to the food or used together with other foods or food ingredients and may appropriately be used according to conventional methods. The mixing amount of the active ingredient may appropriately be determined depending on the purpose of use (prevention or improvement). In general, when producing food or beverages, the composition of the present invention may be added in an amount of preferably 15 wt% or less, more preferably 10 wt% or less, relative to the amounts of the raw materials. However, in the case of long-term intake of the health functional food for the purpose of health, hygiene, or health control, the amount may be below the above range.

In addition to including the above active ingredient, the health functional food of the present invention may include other ingredients as essential ingredients without any particular limitation. For example, as in common beverages, various flavoring agents or natural carbohydrates may be included as additional ingredients. Examples of the natural carbohydrates may be common sugars such as monosaccharides (*e.g*., glucose, fructose, *etc*.), disaccharides such as maltose, sucrose, *etc*.), and polysaccharides (*e.g*., dextrins, cyclodextrins, *etc*.); and sugar alcohols (*e.g*., xylitol, sorbitol, erythritol, *etc*.). As a flavoring agent other than those described above, natural flavoring agents (*e.g*., thaumatin, a stevia extract (*e.g*., rebaudioside A, glycyrrhizin, *etc*.)) and synthetic flavoring agents (*e.g*., saccharin, aspartame, *etc*.) may advantageously be used. The ratio of the natural carbohydrates may appropriately be determined by the selection of those skilled in the art.

In addition to the above, the health functional food of the present invention may include various nutrients, vitamins, minerals (electrolytes), flavoring agents (*e.g*., synthetic and natural flavoring agents, *etc*.), colorants and thickening agents (*e.g*., cheese, chocolate, *etc*.), pectic acid and salts thereof, alginic acid and salts thereof, organic acids, protective colloidal thickeners, pH adjusters, stabilizers, preservatives, glycerin, alcohol, carbonating agents used in carbonated drinks, *etc*. These ingredients may be used independently or in combination, and the proportions of these additives may also appropriately be selected by those skilled in the art.

### 4. Cosmetic composition for preventing or improving inflammatory diseases

Still another aspect of the present invention provides a cosmetic composition for preventing or improving inflammatory diseases, which includes a peptide including the amino acid sequence of SEQ ID NO: 1 as an active ingredient.

Since the peptide including the amino acid sequence of SEQ ID NO: 1 is the same as the peptide described in the "1. Peptides having anti-inflammatory activity" section, for detailed description, the "1. Peptides having anti-inflammatory activity" section is used, hereinafter, only the unique constitution of the cosmetic composition for preventing or improving inflammatory diseases will be described.

As in the pharmaceutical composition, inflammatory diseases may be prevented or improved by inhibiting inflammation, and the cosmetic composition including the peptide of the present invention as an active ingredient, which inhibits inflammation by inhibiting expression or secretion of inflammatory cytokines (*e.g*., TNFα, IL-6, IL-17, IL-1β, IFNγ, *etc*.) or inflammation-related proteins (*e.g*., Cox2, iNos, *etc*.), may effectively be used in preventing or improving inflammatory diseases. In particular, the cosmetic composition of the present invention may effectively be used to prevent or improve inflammatory diseases that occur in the skin, such as inflammatory skin diseases and atopic dermatitis.

The peptide may be included in an amount of 0.001 wt% to 30 wt% of the total 100 wt% of the cosmetic composition, for example, it may be included at 0.1 wt% to 20 wt%, 0.1 wt% to 10 wt%, 1 wt% to 10 wt%, or 2 wt% to 5 wt%, but is not limited thereto.

The cosmetic composition including the peptide of the present invention as an active ingredient may further include, within the range that it does not affect the anti-inflammatory activity of the peptide, for example, other ingredients that have characteristics that can have a synergistic effect on the activity of the peptide. For example, the cosmetic composition may include adjuvants commonly used in the fields of cosmetics or dermatology (*e.g*., fatty substances, organic solvents, solubilizers, thickening and gelling agents, softeners, antioxidants, suspending agents, stabilizers, foaming agents, fragrances, surfactants, water, ionic or non-ionic emulsifiers, fillers, sequestering agents and chelating agents, preservatives, vitamins, blocking agents, humectants, essential oils, dyes, pigments, perfumes, hydrophilic or lipophilic activators, or lipid vesicles) or any other ingredients commonly used in cosmetics, and these ingredients may be included in amounts commonly used in cosmetics or dermatological fields.

The cosmetic composition of the present invention may be prepared in any formulation commonly prepared in the art, for example, may be formulated into cosmetics, such as solutions, suspensions, emulsions, gels, lotions, essences, creams, powders, soaps, shampoos, conditioners, pack masks, surfactant-containing cleanser, cleansing foams, cleansing water, oils, liquid foundations, cream foundations, sprays, *etc.*

When the formulation is a solution or emulsion, as carrier ingredients, a solvent, a solubilizing agent or emulsifying agent may be used, for example, water, ethanol, isopropanol, ethyl carbonate, ethyl acetate, benzyl alcohol, benzyl benzoate, propylene glycol, 1,3-butyl glycol oil, glycerol aliphatic ester, a fatty acid ester of polyethylene glycol or sorbitan, *etc*. may be used. When the formulation of the cosmetic composition is in the form of a suspension, as carrier ingredients, liquid diluents (*e.g*., water, ethanol, propylene glycol, *etc*.), suspending agents (*e.g*., ethoxylated isostearyl alcohol, polyoxyethylene sorbitol ester, polyoxyethylene sorbitan ester, *etc*.), aluminum metahydroxide, microcrystalline cellulose, bentonite, agar, tragacant, *etc*. may be used. When the formulation is a cream or gel, as carrier ingredients, wax, paraffin, tragacant, animal oil, starch, cellulose derivatives, silicone, bentonite, polyethylene glycol, silica, zinc oxide, talc, *etc*. may be used. When the formulation of the cosmetic composition is a powder or spray, as carrier ingredients, silica, talc, an aluminum hydroxyl group, lactose, calcium silicate, or propellants (*e.g*., chlorofluorohydrocarbon, propane/butane, or dimethyl ether) may be included. When the formulation of the cosmetic composition is a surfactant-containing cleanser, as carrier ingredients, aliphatic alcohol sulfate, aliphatic alcohol ether sulfate, sulfosuccinic acid monoester, imidazolinium derivatives, isethionate, methyl taurate, sarcosinate, fatty acid amide ether sulfate, alkylamidobetaine, aliphatic alcohol, fatty acid glyceride, fatty acid diethanolamide, vegetable oils, lanolin derivatives, ethoxylated glycerol fatty acid esters, etc. may be used.

Still another aspect of the present invention provides a method for treating an inflammatory disease, which includes administering a therapeutically effective amount of a peptide including the amino acid sequence of SEQ ID NO: 1 to a subject in need of treatment for the inflammatory disease.

Still another aspect of the present invention provides a method for improving an inflammatory disease, which includes administering a therapeutically effective amount of a peptide including the amino acid sequence of SEQ ID NO: 1 to a subject in need of improvement of the inflammatory disease.

Still another aspect of the present invention provides a use of a peptide including the amino acid sequence of SEQ ID NO: 1 for treating an inflammatory disease.

Still another aspect of the present invention provides a use in the preparation of a drug of a peptide including the amino acid sequence of SEQ ID NO: 1 for treating or preventing inflammatory diseases.

### ADVANTAGEOUS EFFECTS

The peptide provided by the present invention has the effect of inhibiting inflammation thereby inhibiting the expression and secretion of inflammatory cytokines such as TNFα, IL-6, IL-17, IL-1β, and IFNγ, and has the effect of inhibiting the expression of inflammation-related proteins such as Cox2 and iNos. Therefore, the peptide can be effectively used as an active ingredient in a pharmaceutical composition to prevent or treat inflammatory diseases accompanied by inflammation or caused by inflammation, and can be effectively used as an active ingredient in health functional foods or cosmetic compositions to prevent or improve the inflammatory diseases.

However, the effects of the present invention are not limited to those effects mentioned above, and other effects not mentioned above will be clearly understood by those skilled in the art from the following description.

### BRIEF DESCRIPTION OF THE DRAWINGS

FIG. 1 shows the results of a Western blot and a graph thereof, in which inflammation was induced in HaCaT cells by treating with TNFα, and then treated with the peptide of the present invention at a concentration of 5 µM and 50 µM, respectively, to confirm the amount of Cox2 protein. NON refers to the results of the group not treated with TNFα; and NO refers to the results of the group treated with TNFα but not treated with the peptide of the present invention.
FIG. 2 shows the results of a Western blot, in which inflammation was induced in HaCaT cells by treating with LPS, TGFβ, and IL-23, and then treated with the peptide of the present invention at a concentration of 5 µM and 50 µM, respectively, to confirm the amounts of iNos and Cox2 proteins. NON refers to the results of the group not treated with LPS, TGFβ, and IL-23; and NC refers to the results of the group that was treated with LPS, TGFβ, and IL-23 but not treated with the peptide of the present invention.
FIG. 3 shows the results of RT-PCR, in which inflammation was induced in mouse splenocytes by treating with LPS, and then treated with the peptide of the present invention at a concentration of 5 µM and 50 µM, respectively, to confirm the mRNA levels of TNFα and IL-6 genes. NON refers to the results of the group not treated with LPS; and NC refers to the results of the group treated with LPS but not treated with the peptide of the present invention.
FIG. 4 shows the results of RT-PCR, in which inflammation was induced in mouse splenocytes by treating with TNFα, and then treated with the peptide of the present invention at a concentration of 5 µM and 50 µM, respectively, to confirm the mRNA levels of TNFα, Cox2, and IL-1β genes. NON refers to the results of the group not treated with TNFα; and NO refers to the results of the group treated with TNFα but not treated with the peptide of the present invention.
FIG. 5 shows the results of ELISA, in which inflammation was induced in mouse splenocytes by treating with LPS, and then treated with the peptide of the present invention at a concentration of 5 µM and 50 µM, respectively, to confirm the secretion amounts of TNFα, IL-1β, and IFNγ. NON refers to the results of the group not treated with LPS; and NC refers to the results of the group treated with LPS but not treated with the peptide of the present invention.

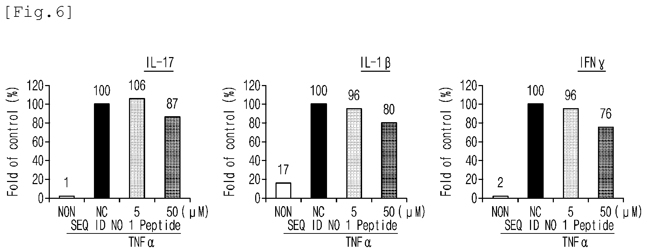

FIG. 6 shows the results of ELISA, in which inflammation was induced in mouse splenocytes by treating with TNFα, and then treated with the peptide of the present invention at a concentration of 5 µM and 50 µM, respectively, to confirm the secretion amounts of IL-17, IL-1β, and IFNγ. NON refers to the results of the group not treated with TNFα; and NC refers to the results of the group treated with TNFα but not treated with the peptide of the present invention.

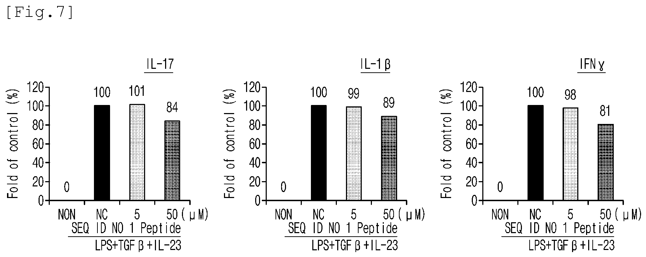

FIG. 7 shows the results of ELISA, in which inflammation was induced in mouse splenocytes by treating with LPS, TGFβ, and IL-23, and then treated with the peptide of the present invention at a concentration of 5 µM and 50 µM, respectively, to confirm the secretion amounts of IL-17, IL-1β, and IFNγ. NON refers to the results of the group not treated with LPS, TGFβ, and IL-23; and NC refers to the results of the group treated with LPS, TGFβ, and IL-23 but not treated with the peptide of the present invention.

### MODE FOR CARRYING OUT THE INVENTION

Hereinafter, the present invention will be described in detail.

However, the following examples specifically illustrate the present invention, and the contents of the present invention are not limited by the following examples.

### Examples

### [Preparation Example] Preparation of peptide

A peptide having the amino acid sequence of SEQ ID NO: 1 shown in Table 1 below was synthesized using an automatic peptide synthesizer (Milligen 9050, Millipore, USA), and the synthesized peptides was purified using the C18 reverse phase high performance liquid chromatography (HPLC) (Waters Associates, USA). The column used was ACQUITY UPLC BEH300 C18 (2.1 mm × 100 mm, 1.7 µm, Waters Co., USA).

**[Table 1]**

| SEQ ID NO | Peptide Sequence |
|---|---|
| 1 | TWNS |

### [Experimental Example 1] Confirmation of decrease in expression level of Cox2 protein after peptide treatment

In order to confirm the anti-inflammatory effect of treatment with the peptides of the present invention having the amino acid sequence of SEQ ID NO: 1, inflammation was induced in HaCaT cells, which are human keratinocytes, and the expression level of Cox2 protein was confirmed. In order to induce inflammation, the cells were treated with TNFα, which is an inflammation-inducing cytokine, and since Cox2 is an enzyme involved in the synthesis of prostaglandins corresponding to an inflammation-related protein, it is possible to confirm whether inflammation is inhibited by measuring the expression level of Cox2.

HaCaT cells were dispensed into a 24-well plate at a concentration of 3 × 10⁵ cells/well and cultured until the next day. The medium was replaced with serum-free medium, and after 4 hours, the cells were treated with TNFα and the peptide of the present invention and cultured again for another 24 hours. In order to induce inflammation, the cells were treated with TNFα at 20 nM, and treated with the peptide at 5 µM and 50 µM, respectively. The HaCaT cells in the group in which inflammation was not induced due to untreatment with TNFα (NON); the negative control group in which the cells were treated with TNFα but not treated with the peptide (NC); and the group in which the cells were treated with TNFα and with the peptide of the present invention at the same concentration as above, respectively, were reacted as described above and recovered to secure the lysates. In order to perform Western blotting, the amount of Cox2 protein was measured using an anti-Cox2 antibody (Cell Signaling Technology, USA). In order to compare the amount of Cox2 protein, the amount of β-actin was also confirmed.

As a result, it was confirmed that when inflammation was induced by treatment with TNFα, the expression level of Cox2 protein was increased showing a more than two-fold increase compared to that of the group not treated with TNFα. In contrast, it was confirmed that when treated with the peptide of the present invention, the amount of Cox2 protein did not increase despite treatment with TNFα, and rather, the expression level of Cox2 was decreased even when compared to that of the group not treated with TNFα (see FIG. 1).

### [Experimental Example 2] Confirmation of decrease in expression level of iNOS/Cox2 proteins after peptide treatment

In order to further confirm the anti-inflammatory effect of the peptide treatment of the present invention in addition to the results of Experimental Example 1, HaCaT cells were treated with Th17 (type 17), which is an inflammation-inducing substance, and the expression levels of inflammation-inducing markers iNos and Cox2 were confirmed. As the inflammation-inducing substances, the cells were treated with LPS, TGFβ, and IL-23, and since iNos is an enzyme involved in the production of nitric oxide (NO), which is an inflammation-related protein induced by LPS, *etc*., it is possible to confirm whether inflammation is inhibited by measuring the expression levels of iNos and Cox2.

HaCaT cells were dispensed into a 24-well plate at a concentration of 3 × 10⁵ cells/well and cultured until the next day. The medium was replaced with serum-free medium, and after 4 hours, the cells were treated with TGFβ, IL-23, and LPS, which are Th17 type inflammation-inducing cytokines, and then with the peptide of the present invention and cultured again for another 24 hours. In order to induce inflammation, the cells were treated with TGFβ at 20 nM/mL, IL-23 at 20 ng/mL, and LPS at 2 µg/mL, and the peptide at 5 µM and 50 µM, respectively. The HaCaT cells in the group in which inflammation was not induced due to untreatment with TGFβ, IL-23, and LPS (NON); the negative control group in which the cells were treated with TGFβ, IL-23, and LPS but not treated with the peptide (NC); and the group in which the cells were treated with TGFβ, IL-23, and LPS and the peptide with the present invention at the same concentration as above, respectively, were reacted as described above and recovered to secure the lysates. In order to perform Western blotting, the amounts of iNos and Cox2 proteins were measured using an anti-iNos antibody and an anti-Cox2 antibody (Cell Signaling Technology, USA). In order to compare the amounts of iNos and Cox2 proteins, the amount of β-actin was also confirmed.

As a result, it was confirmed that when inflammation was induced by treatment with TGFβ, IL-23, and LPS, the expression levels of both iNos and Cox2 proteins were increased compared to those of the groups not treated with TGFβ, IL-23, and LPS, thus showing dark bands. In contrast, it was confirmed that when treated with the peptide of the present invention, the amounts of iNos and Cox2 proteins were not increased despite the treatment with TGFβ, IL-23, and LPS, and the expression levels were decreased compared to those of the groups not treated with the peptide (see FIG. 2).

### [Experimental Example 3] Confirmation of decrease in expression level of inflammation-promoting cytokine genes after peptide treatment

### <3-1> Confirmation of mRNA expression level of inflammatory cytokines in cells with inflammation induced by LPS

Inflammation was induced in mouse splenocytes, and the expression levels of TNFα, and IL-6 genes, which is the inflammatory cytokines were confirmed. In order to induce inflammation, the cells were treated with LPS, an inflammation-inducing antigen, and since TNFα corresponds to a signaling protein associated with inflammation, and IL-6 corresponds to a cytokine that promotes inflammation, the degree of inflammation can be confirmed by measuring the expression levels of TNFα and IL-6 genes.

Mouse splenocytes were dispensed into a 24-well plate at a concentration of 1 × 10⁷ cells/well and cultured until the next day. The medium was replaced with serum-free medium, and after 3 hours, the cells were treated with LPS and the peptide of the present invention and then cultured again for another 24 hours. In order to induce inflammation, the cells were treated with LPS at 2 µg/mL, and the peptide at 5 µM and 50 µM, respectively. The mouse splenocytes in the group in which inflammation was not induced due to untreatment with LPS (NON); the negative control group in which the cells were treated with LPS but not treated with the peptide (NC); and the group in which the cells were treated with LPS along with the peptide of the present invention at the same concentration as above, respectively, were reacted as described above and recovered to isolate the RNA. After quantifying the amount of isolated RNA, cDNA was synthesized using a cDNA synthesis kit (Intron, Korea), PCR was performed using a PCR premix (Intron, Korea) and primers for TNFα, and IL-6. As the primer sequences for TNFα and IL-6, those having the sequences shown in Table 2 below were used. After performing the PCR, each sample was loaded onto a 5% agarose gel to measure its mRNA expression level. In order to compare the amount of mRNA, the amount of GADPH mRNA was also confirmed.

**[Table 2]**

| SEQ ID NO | Sequence Name | Nucleotide Sequence |
|---|---|---|
| 2 | TNFα forward primer | 5'- AACATCCAACCTTCCCAAACG -3' |
| 3 | TNFα reverse primer | 5'- GACCCTAAGCCCCCAATTCTC -3' |
| 4 | IL-6 forward primer | 5'- GGAACGGTATCCACGGATGT -3' |
| 5 | IL-6 reverse primer | 5'- GAGGTACATCACGTGGGAAG -3' |

As a result, it was confirmed that when inflammation was induced by treatment with LPS, the mRNA levels of TNFα and IL-6 were increased compared to those of the groups not treated with LPS. In contrast, it was confirmed that when treated with the peptide of the present invention, the mRNA expression levels of TNFα and IL-6 were not increased despite treatment with LPS, and the expression levels were decreased compared to those of the groups not treated with the peptide (see FIG. 3).

### <3-2> Confirmation of mRNA expression level of inflammatory cytokines in cells with inflammation induced by with TNFα

In order to further confirm the anti-inflammatory effect of the peptide treatment of the present invention in addition to the results of Experimental Example <3-1>, inflammation was induced by treating mouse splenocytes with TNFα, and the expression levels of inflammatory cytokines TNFα and IL-1β, and Cox2, which is an inflammation-inducing marker, genes were confirmed. In order to induce inflammation, the cells were treated with cytokine TNFα, and since TNFα corresponds to a signaling protein associated with inflammation, IL-1β corresponds to a cytokine that promotes inflammation, and Cox2 is a protein associated with inflammation, the degree of inflammation can be confirmed by measuring the expression levels of TNFα, Cox2, and IL-1β genes.

Mouse splenocytes were dispensed into a 24-well plate at a concentration of 1 × 10⁷ cells/well and cultured until the next day. The medium was replaced with serum-free medium, and after 3 hours, the cells were treated with TNFα and the peptide of the present invention and then cultured for another 3 hours. In order to induce inflammation, the cells were treated with TNFα at 20 nM, and the peptide at 5 µM and 50 µM, respectively. The splenocytes in the group in which inflammation was not induced due to untreatment with TNFα (NON); the negative control group in which the cells were treated with TNFα but not treated with the peptide (NC); and the group in which the cells were treated with TNFα along with the peptide of the present invention at the same concentration as above, respectively, were reacted as described above and recovered to isolate the RNA. After quantifying the amount of isolated RNA, cDNA was synthesized using a cDNA synthesis kit (Intron, Korea), PCR was performed using a PCR premix (Intron, Korea) and primers for TNFα, Cox2, and IL-1β. As the primer sequences for TNFα, those having the sequences shown in Table 2 above were used, and as the primer sequences for Cox2 and IL-1β, those having the sequences shown in Table 3 below were used. After performing the PCR, each sample was loaded onto a 5% agarose gel to measure its mRNA expression level. In order to compare the amount of mRNA, the amount of GADPH mRNA was also confirmed.

**[Table 3]**

| SEQ ID NO | Sequence Name | Nucleotide Sequence |
|---|---|---|
| 6 | Cox2 forward primer | 5'- TGAGTGGTAGCCAGCAAAGC -3' |
| 7 | Cox2 reverse primer | 5'- CTGCAGTCCAGGTTCAATGG -3' |
| 8 | IL-1β forward primer | 5'- TTCGACACATGGGATAACGA -3' |
| 9 | IL-1β reverse primer | 5'- TCTTTCAACACGCAGGACAG -3' |

As a result, it was confirmed that when inflammation was induced by treatment with TNFα, the mRNA levels of TNFα, Cox2, and IL-1β were increased compared to those of the groups not treated with TNFα. In contrast, it was confirmed that when treated with the peptide of the present invention, the mRNA expression levels of TNFα, Cox2, and IL-1β were not increased despite treatment with TNFα, and the expression levels were decreased compared to those of the groups not treated with the peptide (see FIG. 4).

### [Experimental Example 4] Confirmation of decrease in secretion amount of inflammation-promoting cytokines after peptide treatment

### <4-1> Confirmation of secretion amounts of inflammatory cytokines in cells with inflammation induced by LPS

Inflammation was induced in mouse splenocytes, and the secretion amounts of inflammatory cytokines TNFα, IL-1β, and IFNγ were confirmed. In order to induce inflammation, the cells were treated with an inflammation-inducing antigen, LPS, and since TNFα corresponds to a signaling protein associated with inflammation, IL-1β corresponds to a cytokine that promotes inflammation, and IFNγ also corresponds to a cytokine that promotes inflammation, the degree of inflammation can be confirmed by measuring the secretion amounts of TNFα, IL-1β, and IFNγ.

Mouse splenocytes were dispensed into a 24-well plate at a concentration of 1 × 10⁷ cells/well and cultured until the next day. The medium was replaced with serum-free medium, and after 4 hours, the cells were treated with LPS and the peptide of the present invention and then cultured again for another 24 hours. In order to induce inflammation, the cells were treated with LPS at 2 µg/mL, and the peptide at 5 µM and 50 µM, respectively. The mouse splenocytes in the group in which inflammation was not induced due to untreatment with LPS (NON); the negative control group in which the cells were treated with LPS but not treated with the peptide (NC); and the group in which the cells were treated with LPS along with the peptide of the present invention at the same concentration as above, respectively, were reacted as described above and the cell cultures were recovered. For the recovered cell cultures, the secretion amounts of TNFα, IL-1β, and IFNγ were confirmed through the ELISA technique using the TNF-alpha Quantikine ELISA Kit (R&D system, USA), IL-1 beta Quantikine ELISA Kit (R&D system, USA), and IFN-gamma Quantikine ELISA Kit (R&D system, USA).

As a result, it was confirmed that when inflammation was induced by treatment with LPS, the secretion amounts of TNFα, IL-1β, and IFNγ were significantly increased compared to those of the groups not treated with LPS. When treated with the peptide of the present invention, it was confirmed that the secretion amounts of TNFα, IL-1β, and IFNγ were decreased despite the treatment with LPS (see FIG. 5).

### <4-2> Confirmation of secretion amounts of inflammatory cytokines in cells with inflammation induced by TNFα

Inflammation was induced by treating mouse splenocytes with the inflammatory cytokine TNFα, and the secretion amounts of the inflammatory cytokines IL-17, IL-1β, and IFNγ were confirmed. IL-17 corresponds to a pro-inflammatory cytokine, and the descriptions of IL-1β and IFNγ are the same as above.

Mouse splenocytes were dispensed into a 24-well plate at a concentration of 1 × 10⁷ cells/well and cultured until the next day. The medium was replaced with serum-free medium, and after 4 hours, the cells were treated with TNFα and the peptide of the present invention and then cultured again for another 24 hours. In order to induce inflammation, the cells were treated with TNFα at 20 nM, and the peptide at 5 µM and 50 µM, respectively. The mouse splenocytes in the group in which inflammation was not induced due to untreatment with TNFα (NON); the negative control group in which the cells were treated with TNFα but not treated with the peptide (NC); and the group in which the cells were treated with TNFα along with the peptide of the present invention at the same concentration as above, respectively, were reacted as described above and the cell cultures were recovered. For the recovered cell cultures, the secretion amounts of IL-17, IL-1β, and IFNγ were confirmed through the ELISA technique using the IL-17 Quantikine ELISA Kit (R&D system, USA), IL-1 beta Quantikine ELISA Kit (R&D system, USA), and IFN-gamma Quantikine ELISA Kit (R&D system, USA).

As a result, it was confirmed that when inflammation was induced by treatment with TNFα, the secretion amounts of IL-17, IL-1β, and IFNγ were significantly increased compared to those of the groups not treated with TNFα. When treated with the peptide of the present invention at 5 µM, the secretion amount of IL-17 was slightly increased, but the secretion amounts of IL-1β and IFNγ were decreased, whereas when the amount was increased by treating with the peptide at an increased concentration of 50 µM, the secretion amounts of IL-17, IL-1β, and IFNγ were all decreased, thus confirming the effect of the peptide of the present invention on reducing the secretion amounts of inflammation-inducing markers according to its concentration (see FIG. 6).

### <4-3> Confirmation of secretion amounts of inflammatory cytokines in cells with inflammation induced by LPS, TGFβ, and IL-23

Inflammation was induced by treating mouse splenocytes with LPS, which is an inflammation-inducing antigen, and TGFβ and IL-23, which are inflammatory cytokines, and the secretion amounts of the inflammatory cytokines IL-17, IL-1β, and IFNγ were confirmed. The descriptions of IL-17, IL-1β, and IFNγ are the same as above.

Mouse splenocytes were dispensed into a 24-well plate at a concentration of 1 × 10⁷ cells/well and cultured until the next day. The medium was replaced with serum-free medium, and after 4 hours, the cells were treated with LPS, TGFβ, and IL-23 and the peptide of the present invention and then cultured again for another 24 hours. In order to induce inflammation, the cells were treated with LPS at 2 µg/mL, TGFβ at 20 ng/mL, IL-23 at 20 ng/mL, and the peptide at 5 µM and 50 µM, respectively. The mouse splenocytes in the group in which inflammation was not induced due to untreatment with LPS, TGFβ, and IL-23 (NON); the negative control group in which the cells were treated with LPS, TGFβ, and IL-23 but not treated with the peptide (NC); and the group in which the cells were treated with LPS, TGFβ, and IL-23 along with the peptide of the present invention at the same concentration as above, respectively, were reacted as described above and the cell cultures were recovered. For the recovered cell cultures, the secretion amounts of IL-17, IL-1β, and IFNγ were confirmed through the ELISA technique using the IL-17 Quantikine ELISA Kit (R&D system, USA), IL-1 beta Quantikine ELISA Kit (R&D system, USA), IFN-gamma Quantikine ELISA Kit (R&D system, USA).

As a result, it was confirmed that when inflammation was induced by treatment with LPS, TGFβ, and IL-23, the secretion amounts of IL-17, IL-1β, and IFNγ were significantly increased compared to those of the groups not treated with LPS, TGFβ, and IL-23. When treated with the peptide of the present invention at 5 µM, the secretion amount of IL-17 was slightly increased, but the secretion amounts of IL-1β and IFNγ were decreased, whereas when the amount was increased by treating with the peptide at an increased concentration of 50 µM, the secretion amounts of IL-17, IL-1β, and IFNγ were all decreased, thus confirming the effect of the peptide of the present invention on reducing the secretion amounts of inflammation-inducing markers according to its concentration (see FIG. 7).

Considering the results of the Experimental Examples above, even in cells where inflammation was induced, treatment with the peptide of the present invention reduced the amounts of proteins or expression levels of genes associated with inflammation, and reduced expression and secretion of cytokines that promote inflammation, thus confirming the effect of the peptide of the present invention on inhibiting inflammation. In addition, when the peptide of the present invention was treated with a higher amount, the above effect appeared to be more apparent, thus confirming that the inhibitory effect on inflammation shown in the results of Experimental Examples above are due to the peptide of the present invention.

From the above, the present invention has been described in detail only with respect to the described embodiments, but it is obvious to those skilled in the art that various changes and modifications are possible within the technical scope of the present invention, and it is natural that such changes and modifications fall within the scope of the appended claims.

## Claims

1. A peptide having anti-inflammatory activity, comprising the amino acid sequence of SEQ ID NO: 1.

2. The peptide of claim 1, wherein the peptide inhibits the expression of inflammatory cytokines.

3. The peptide of claim 2, wherein the inflammatory cytokine is one or more selected from the group consisting of TNFα, IL-6, IL-17, IL-1β, and IFNγ.

4. The peptide of claim 1, wherein the peptide inhibits the expression of Cox2.

5. The peptide of claim 1, wherein the peptide inhibits the expression of iNos.

6. A pharmaceutical composition for preventing or treating inflammatory diseases, comprising the peptide of any one of claims 1 to 5 as an active ingredient.

7. The pharmaceutical composition of claim 6, wherein the inflammatory diseases is one or more selected from the group consisting of rhinitis, bronchitis, periodontitis, pancreatitis, gastritis, gastric ulcer, inflammatory skin disease, atopic dermatitis, encephilitis, sepsis, inflammatory enteritis, chronic obstructive pulmonary disease, septic shock, pulmonary fibrosis, undifferentiated spondyloarthropathy, undifferentiated arthropathy, arthritis, inflammatory osteolysis, chronic inflammatory disease caused by chronic viral or bacterial infection, colitis, inflammatory bowel disease, type 1 diabetes, rheumatoid arthritis, reactive arthritis, osteoarthritis, psoriasis, scleroderma, osteoporosis, atherosclerosis, myocarditis, endocarditis, pericarditis, cystic fibrosis, Hashimoto's thyroiditis, Graves' disease, leprosy, syphilis, lyme, borreliosis, neuroborreliosis, tuberculosis, sarcoidosis, lupus, discoid lupus, chilblains lupus, lupus nephritis, systemic lupus erythematosus, macular degeneration, uveitis, irritable bowel syndrome, Crohn's disease, Sjögren's syndrome, fibromyalgia, chronic fatigue syndrome, chronic fatigue immune dysfunction syndrome, myalgic encephalomyelitis, amyotrophic lateral sclerosis, Parkinson's disease and multiple sclerosis.

8. A health functional food for preventing or improving inflammatory diseases, comprising the peptide of any one of claims 1 to 5 as an active ingredient.

9. A cosmetic composition for preventing or improving inflammatory diseases, comprising the peptide of any one of claims 1 to 5 as an active ingredient.

10. The cosmetic composition of claim 9, wherein the cosmetic composition has one type of formulation selected from the group consisting of solutions, suspensions, emulsions, gels, lotions, essences, creams, powders, soaps, shampoos, conditioners, pack masks, surfactant-containing cleansers, cleansing foams, cleansing waters, oils, liquid foundations, cream foundations, and sprays.
